(19) Europäisches Patentamt
European Patent Office
Office européen des brevets

(11) Veröffentlichungsnummer: **0 257 448 B1**

(12) # EUROPÄISCHE PATENTSCHRIFT

(45) Veröffentlichungstag der Patentschrift: **11.03.92**

(51) Int. Cl.5: **C07C 233/59**, A01N 53/00, C07C 231/02, C07C 231/00

(21) Anmeldenummer: **87111659.6**

(22) Anmeldetag: **12.08.87**

Die Akte enthält technische Angaben, die nach dem Eingang der Anmeldung eingereicht wurden und die nicht in dieser Patentschrift enthalten sind.

(54) **Optisch aktive Cyclopropancarboxamide.**

(30) Priorität: **25.08.86 JP 197357/87**

(43) Veröffentlichungstag der Anmeldung:
**02.03.88 Patentblatt 88/09**

(45) Bekanntmachung des Hinweises auf die Patenterteilung:
**11.03.92 Patentblatt 92/11**

(84) Benannte Vertragsstaaten:
**BE CH DE FR GB IT LI NL**

(56) Entgegenhaltungen:
**EP-A- 0 170 842**

**Monosaccharide Chemistry, Penguin Books 1972, page 21**

(73) Patentinhaber: **NIHON BAYER AGROCHEM K.K.**
**7-1, Nihonbashi Honcho 2-chome Chuo-ku**
**Tokyo 103(JP)**

(72) Erfinder: **Kurahashi, Yoshio**
**47-15, Oya-machi**
**Hachioji-shi Tokyo(JP)**
Erfinder: **Kagabu, Shinzo**
**1768-532, Sagiyama**
**Gifu-shi Gifu-ken(JP)**
Erfinder: **Matsumoto, Noboru**
**39-15, Namiki-cho**
**Hachioji-shi Tokyo(JP)**
Erfinder: **Yamada, Takayo**
**2-16-2, Koyasu-machi**
**Hachioji-shi Tokyo(JP)**
Erfinder: **Wada, Katsuaki**
**39-15, Namiki-cho**
**Hachioji-shi Tokyo(JP)**
Erfinder: **Kondo, Toshihito**
**39-15, Namiki-cho**
**Hachioji-shi Tokyo(JP)**

(74) Vertreter: **Schumacher, Günter, Dr. et al**
**c/o Bayer AG Konzernverwaltung RP Patentabteilung**
**W-5090 Leverkusen 1 Bayerwerk(DE)**

EP 0 257 448 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue, optisch aktive Cyclopropancarboxamide, ein Verfahren zu ihrer Herstellung und ihre Verwendung als landwirtschaftliche Fungizide.

Es wurde bereits offenbart, daß bestimmte N-Benzylcyclopropancarboxamide Aktivitäten als landwirtschaftliche Fungizide aufweisen (siehe die JP-OS 15867/1986).

Gefunden wurden nunmehr neue, optisch aktive Cyclopropancarboxamide der Formel (I)

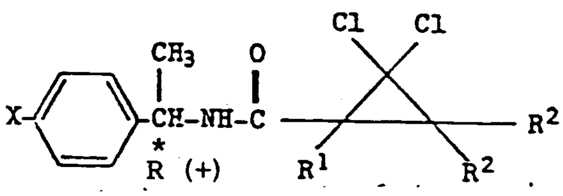

(I)

in welcher die Amin-Struktureinheit in der R (+)-Konfiguration vorliegt und in der

X Halogen bezeichnet,

$R^1$ Wasserstoff oder Alkyl bezeichnet und die

$R^2$s für Wasserstoff oder Alkyl stehen.

Die Verbindungen der Formel (I) werden mit Hilfe eines Verfahrens hergestellt, bei dem

a) Verbindungen der Formel (II)

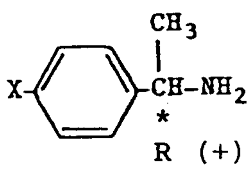

(II)

(in der X die oben angegebene Bedeutung hat) mit Verbindungen der Formel (III)

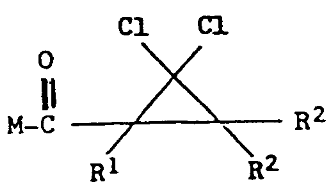

(III)

(in der $R^1$ und $R^2$ die oben angegebenen Bedeutungen haben und M für Hydroxyl oder ein Halogen steht), in Gegenwart eines inerten Lösungsmittels und gegebenenfalls in Gegenwart eines säurebindenden Mittels umgesetzt werden oder

b) optisch aktive Verbindungen der Formel (I), bei denen die Amin-Struktureinheit optisch aktiv ist, einer physikalischen Behandlung unterzogen werden.

Die Verbindungen der Formel (I) gemäß der Erfindung besitzen starke fungizide Wirkung.

Überraschenderweise zeigen die vorliegenden Verbindungen der Formel (I) eine fungizide Wirkung, die wesentlich stärker ist als diejenige der bekannten Verbindungen, die in der oben bezeichneten JP-OS 15867/1986 offenbart sind, wobei die bekannten Verbindungen die gleiche Formel wie die Formel (I) haben und eine Struktur besitzen, in der sowohl die Säure-Struktureinheit als auch die Amin-Struktureinheit in racemischer Konfiguration vorliegen. Die Verbindungen gemäß der Erfindung zeigen eine starke fungizide Wirkung gegenüber pathogenen Fungi, insbesondere gegen die durch Piricularia oryzae verursachte Brusone-Krankheit von Reis.

Die Ziele und die verschiedenen Vorteile der vorliegenden Erfindung gehen im einzelnen aus der folgenden Beschreibung hervor.

In der Formel (I) bezeichnet

X Halogen, nämlich Fluor, Chlor, Brom oder Iod;
$R^1$ Wasserstoff oder einen Alkyl-Rest mit 1 bis 6 Kohlenstoffatomen wie Methyl, Ethyl, n- oder iso-Propyl, n-, iso-, sec- oder tert-Butyl, n-Pentyl oder n-Hexyl und die
$R^2$s Wasserstoff oder Alkyl mit 1 bis 6 Kohlenstoffatomen.

Vorzugsweise bezeichnen in der Formel (I)
X Chlor oder Brom,
$R^1$ einen Alkyl-Rest mit 1 bis 4 Kohlenstoff-Atomen und die
$R^2$s Wasserstoff oder einen Alkyl-Rest mit 1 bis 4 Kohlenstoff-Atomen.

Besonders bevorzugt bezeichnen in der Formel (I)
X Chlor oder Brom,
$R^1$ Methyl, Ethyl oder Isopropyl und die
$R^2$s Wasserstoff oder Methyl.

Für die optisch aktiven Verbindungen der Formel (I) wird eine Struktur bevorzugt, in der die Amin-Struktureinheit in der R (+)-Konfiguration vorliegt und die Säure-Struktureinheit in der racemischen Konfiguration vorliegt. Besonders bevorzugt hat die Amin-Struktureinheit die Struktur eines Spiegelbild-Isomers mit R (+)-Konfiguration.

Beispiele für die Verbindungen der Formel (I) gemäß der Erfindung sind im Folgenden aufgeführt.

| 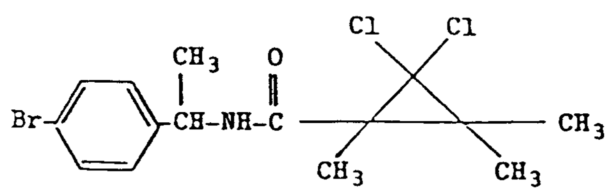 | |
|---|---|
| Verbindung (a) | Epimere, worin die Amin-Struktureinheit in der R(+)-Konfiguration vorliegt; $[\alpha]_D^{21}$ +61,2° (c = 1,02 g/100 ml Ethanol) |
| Verbindung (b) | Diastereomer, worin die Amin-Struktureinheit in der R(+)-Konfiguration vorliegt; $[\alpha]_D^{21}$ +64,2° (c = 1,13 g/100 ml Ethanol) |

| | |
|---|---|
| 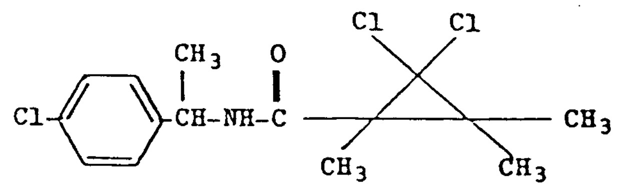 | |
| Verbindung (d) | Epimere, worin die Amin-Struktureinheit in der R(+)-Konfiguration vorliegt |
| Verbindung (e) | Diastereomer, worin die Amin-Struktureinheit in der R(+)-Konfiguration vorliegt |

Wenn die in dem Verfahren a) eingesetzten Ausgangsstoffe R(+)-4-Bromo-α-phenethylamin und 1,3,3-Trimethyl-2,2-dichlorocyclopropancarbonsäurechlorid sind, kann die Reaktion durch das folgende Schema veranschaulicht werden:

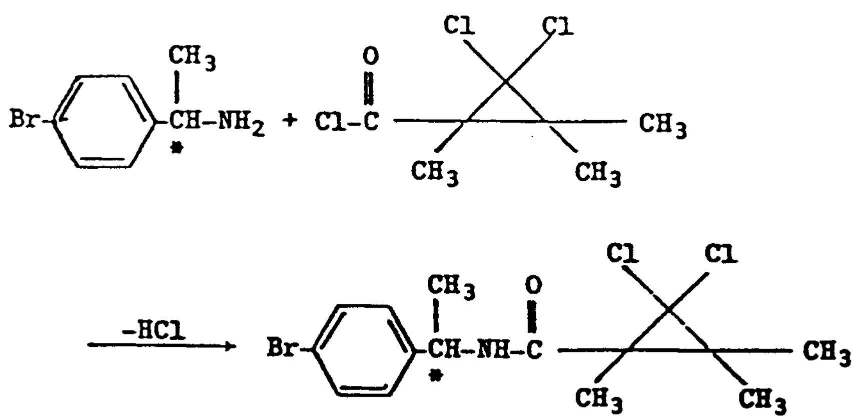


(Amin-Struktureinheit: R(+); Säure-Struktureinheit: ±)

In der Formel (II) hat X die im Vorstehenden angegebene Bedeutung und bezeichnet vorzugsweise die gleichen Reste wie diejenigen, die in der Formel (I) bevorzugt sind.

Einige der Ausgangs-Verbindungen der Formel (II) sind auf den Gebieten der organischen Chemie bereits bekannt. Beispiele für die Verbindungen der Formel (II) sind

R(+)-4-Bromo-α-phenethylamin und
R(+)-4-Chloro-α-phenethylamin.

In den Ausgangs-Verbindungen der Formel (III) haben $R^1$, $R^2$ und M die oben angegebenen Bedeutungen. Vorzugsweise haben $R^1$ und $R^2$ in der Formel (III) die gleichen Bedeutungen wie diejenigen, die in der Formel (I) bevorzugt sind. M bezeichnet vorzugsweise Hydroxyl oder Chlor.

Einige Verbindungen der Formel (III) sind bekannt und in der JP-OS 15867/1986 beschrieben. Beispiele

für diese Verbindungen sind 1,3,3-Trimethyl-2,2-dichlorocyclopropancarbonsäure und ihr Chlorid.

Bei der Durchführung des Verfahrens (a) können beliebige inerte organische Lösungsmittel als Verdünnungsmittel verwendet werden. Beispiele für die Lösungsmittel sind aliphatische, cycloaliphatische und aromatische, gegebenenfalls chlorierte, Kohlenwasserstoff wie Hexan, Cyclohexan, Petrolether, Ligroin, Benzol, Toluol, Xylol, Methylenchlorid, Chloroform, Kohlenstofftetrachlorid, Chlorbenzol, Ethylenchlorid, und Trichloroethylen; Ether, zum Beispiel Diethylether, Methylethylether, Di-isopropylether, Dibutylether, Propylenoxid, Dioxan und Tetrahydrofuran; Ketone, zum Beispiel Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Wenn die Ausgangsstoffe der Formel (III) die Carbonsäure sind, kann das Verfahren (a) in Gegenwart eines die Wasserabspaltung-Kondensation fördernden Mittels, beispielsweise von N,N'-Dicyclohexylcarbodiimid, durchgeführt werden.

Wenn die Ausgangsstoffe der Formel (III) die Säurehalogenide sind, kann das Verfahren (a) in Gegenwart eines säurebindenden Mittels durchgeführt werden.

Beispiele für säurebindende Mittel sind Hydroxide, Carbonate, Hydrogencarbonate und Alkoholate der Alkalimetalle und tertiäre Amine wie Triethylamin, Diethylanilin, Pyridin und dergleichen.

Bei dem Verfahren (a) kann die Reaktionstemperatur innerhalb eines weiten Temperaturbereichs variiert werden. Beispielsweise kann das Verfahren (a) bei einer Temperatur von etwa -20 °C bis zum Siedepunkt der Reaktionsmischung, vorzugsweise bei einer Temperatur von etwa 0 °C bis 100 °C, durchgeführt werden. Obwohl das Verfahren (a) vorzugsweise unter normalem Druck durchgeführt werden kann, ist es auch möglich, einen höheren oder neidrigeren Druck anzuwenden.

Beim Verfahren (a) ist es möglich, etwa 1 mol oder eine geringe Überschußmenge der Verbindung der Formel (III) auf 1 mol der Verbindung der Formel (II) einzusetzen. Diese Ausgangs-Verbindungen können miteinander in einem inerten Lösungsmittel umgesetzt werden, um eine angestrebte optisch aktive Verbindung der Formel (I) zu erhalten.

In dem Verfahren (b) ist es möglich, ein angestrebtes Diastereomer mit Hilfe der Säulenchromatographie zu erhalten.

Ein bevorzugtes Enantiomer der Formel (I) kann in einfacher Weise dadurch erhalten werden, daß ein entsprechendes Epimer, worin die Amin-Struktureinheit die R(+)-Konfiguration aufweist, einer säulenchromatographischen Operation unterzogen wird.

Die aktiven Verbindungen gemäß der Erfindung zeigen eine potente fungizide und bakterizide Wirkung, so daß die Verbindungen zur Bekämpfung unerwünschter pathogener Fungi und Bakterien eingesetzt werden können.

Die aktiven Verbindungen gemäß der Erfindung zeigen eine potente mikrobizide Wirkung und können in der Praxis zur Bekämpfung unerwünschter Mikroorganismen eingesetzt werden. Die aktiven Verbindungen sind zur Verwendung als Pflanzenschutzmittel geeignet.

Fungizide Mittel werden im Pflanzenschutz beispielsweise eingesetzt zur Bekämpfung von
Plasmodiophoromycetes,
Oomycetes,
Chytridiomycetes,
Zygomycetes,
Ascomycetes,
Basidiomycetes und
Deuteromycetes.

Bakterizide Mittel werden im Pflanzenschutz beispielsweise eingesetzt zur Bekämpfung von
Pseudomonadaceae,
Rhizobiaceae,
Enterobacteriaceae,
Corynebacteriaceae und
Streptomycetaceae.

Einige der Organismen, die fungale und bakterielle Erkrankungen verursachen und unter die oben aufgeführten Sammelbegriffe fallen, seien im Folgenden als nichtbeschränkende Beispiele genannt:
Species Xanthomonas
wie beispielsweise Xanthomonas campestris pv. oryzae;
Species Pseudomonas
wie beispielsweise Pseudomonas syringae pv. lacrymans;

Species Erwinia
wie beispielsweise Erwinia amylovora;
Species Pythium
wie beispielsweise Pythium ultimum;
Species Phytophthora
wie beispielsweise Phytophthora infestans;
Species Pseudoperonospora
wie beispielsweise Pseudoperonospora cubensis;
Species Plasmopara
wie beispielsweise Plasmopara viticola;
Species Peronospora
wie beispielsweise Peronospora pisi oder P. brassicae;
Species Erysiphe
wie beispielsweise Erysiphe graminis;
Species Sphaerotheca
wie beispielsweise Sphaerotheca fuliginea;
Species Podosphaera
wie beispielsweise Podosphaera leucotricha;
Species Venturia
wie beispielsweise Venturia inaequalis;
Species Pyrenophora
wie beispielsweise Pyrenophora teres oder P. graminea (Conidiale Form: Drechslera, Synonym: Helminthosporium);
Species Cochliobolus
wie beispielsweise Cochliobolus sativus (Conidiale Form: Drechslera, Synonym: Helminthosporium);
Species Uromyces
wie beispielsweise Uromyces appendiculatus;
Species Puccinia
wie beispielsweise Puccinia recondita;
Species Tilletia
wie beispielsweise Tilletia caries;
Species Ustilago
wie beispielsweise Ustilago nuda oder Ustilago avenae;
Species Pellicularia
wie beispielsweise Pellicularia sasakii;
Species Piricularia
wie beispielsweise Piricularia oryzae;
Species Fusarium
wie beispielsweise Fusarium culmorum;
Species Botrytis
wie beispielsweise Botrytis cinerea;
Species Septoria
wie beispielsweise Septoria nodorum;
Species Leptosphaeria
wie beispielsweise Leptosphaeria nodorum;
Species Cercospora
wie beispielsweise Cercospora canescens;
Species Alternaria
wie beispielsweise Alternaria brassicae;
Species Pseudocercosporella
wie beispielsweise Pseudocercosporella herpotrichoides.

Insbesondere zeigen die aktiven Verbindungen gemäß der Erfindung eine sehr potente fungizide Wirkung gegen den die Brusone-Krankheit von Reis verursachenden Pilz (Piricularia oryzae).

Die gute Tolerierung der aktiven Verbindungen durch die Pflanzen bei den zur Bekämpfung der Pflanzenerkrankungen erforderlichen Konzentrationen erlaubt die Behandlung der oberirdischen Pflanzenteile, der Teile der vegetativen Fortpflanzung und der Samen sowie des Bodens.

Die erfindungsgemäßen aktiven Verbindungen haben sehr niedrige Werte der Toxizität gegenüber warmblütigen Tieren, so daß die Verbindungen sicher verwendet werden können.

Die Aktiven Verbindungen können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche und synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucheropatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe und ebenso auch Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischen Materialien wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-%, der aktiven Verbindung.

Die aktiven Verbindungen gemäß der vorliegenden Erfindung können in ihren Formulierungen oder ihren verschiedenen Anwendungsformen als Gemische mit anderen bekannten aktiven Verbindungen vorliegen, etwa mit Fungiziden, Bakteriziden, Insektiziden, Akariziden, Nematiziden, Herbiziden, vogelabweisenden Mitteln, Wachstumsfaktoren, Pflanzennährstoffen und Mitteln zur Verbesserung der Bodenstruktur.

Die aktiven Verbindungen können als solche eingesetzt werden, oder sie können in Form solcher Formulierungen oder Anwendungsformen, die daraus durch weitere Verdünnung hergestellt werden, etwa gebrauchsfertigen Lösungen, Emulsionen, Suspensionen, Pulvern, Pasten und Granulat, eingesetzt werden. Sie werden in üblicher Weise ausgebracht, etwa durch Bewässern, Eintauchen, Spritzen, Zerstäuben, Vernebeln, Verdampfen, Einspritzen, Bilden von Aufschlämmungen, Aufpinseln, Stäuben, Streuen, Trockenbedecken, Feuchtbedecken, Naßbedecken, Schlammbedecken und Umhüllen mit einer Kruste.

Bei der Behandlung von Pflanzenteilen können die Konzentrationen der aktiven Verbindung in den Anwendungsformen innerhalb eines beträchtlichen Bereichs variiert werden. Sie liegen im allgemeinen in einem Bereich von 1 bis 0,0001 Gew.-%, und vorzugsweise von 0,5 bis 0,001 Gew.-%.

Im Fall der Saatgut-Behandlung werden Mengen der aktiven Verbindung von beispielsweise 0,001 bis 50 g, vorzugsweise 0,01 bis 10 g, auf 1 kg des Saatguts zur Anwendung gebracht.

Zur Behandlung des Bodens werden im allgemeinen am Wirkungsort Konzentrationen der aktiven Verbindung von 0,00001 bis 0,1 Gew.-%, insbesondere 0,0001 bis etwa 0,02 Gew,-%, eingesetzt.

Die Herstellung und die Verwendung der aktiven Verbindungen gemäß der Erfindung ist den folgenden Beispielen zu entnehmen.

Herstellungsbeispiele

Beispiel 1

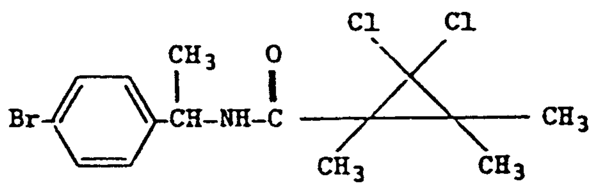

**(Amin-Struktureinheit: R(+); Säure-Struktureinheit: ±)**

**(Verbindung Nr. 1)**

4,0 g R(+)-4-Bromo-α-methylbenzylamin und 2,2 g Triethylamin werden in 30 ml Tetrahydrofuran gelöst und die erhaltene Lösung wird mit Eis gekühlt. Zu dieser Lösung wird portionsweise eine Lösung von 4,28 g 1,3,3-Trimethyl-2,2-dichlorocyclopropancarbonsäurechlorid in 20 ml Tetrahydrofuran unter Rühren hinzugefügt. Nach der Zugabe wird die Reaktionsmischung auf Raumtemperatur gebracht, 5 h gerührt, auf 60 °C erhitzt und dann weitere 2 h gerührt. Das Tetrahydrofuran wird abdestilliert, und der Rückstand wird mit 50 ml Chloroform vermischt. Die Reaktionsmischung wird mit den folgenden Flüssigkeiten in der angegebenen Reihenfolge gewaschen: Wasser, verdünnte Salzsäure, verdünnte wäßrige Natriumhydrogencarbonat-Lösung und wiederum Wasser. Die Chloroform-Schicht wird über wasserfreiem Natriumsulfat getrocknet. Das Chloroform wird unter vermindertem Druck abdestilliert und der Rückstand wird aus einem Lösungsmittel-Gemisch aus 7 Teilen n-Hexan und 3 Teilen Ether umkristallisiert, so daß 8,38 g des angestrebten Epimers, d.h. R(+)-4-Bromo-α-methylbenzylamid der (±)-1,3,3-Trimethyl-2,2-dichlorocyclopropancarbonsäure, erhalten werden.
Schmp. 148-159 °C.
$[\alpha]_D^{21}$ +61,2 ° (C = 1,02 g/100 ml Ethanol)

Die analoge Arbeitsweise kann zur Umsetzung von R(+)-4-Chloro-α-methylbenzylamin mit 1,3,3-Trimethyl-2,2-dichlorocyclopropancarbonsäurechlorid angewandt werden, so daß das angestrebte Epimer, d.h. R(+)-4-Chloro-α-methylbenzylamid der (±)-1,3,3-Trimethyl-2,2-dichlorocyclopropancarbonsäure, erhalten werden kann.

Beispiel 2

7,0 g der in Beispiel 1 erhaltenen Verbindung werden einer säulenchromatographischen Operation unterzogen, so daß 3,48 g des angestrebten Diastereomers aus der ersten Fraktion erhalten wird. Dieses Isomer wird als farbloses kristallines Material mit einem Schmp. 147-148 °C und einer spezifischen Drehung $[\alpha]_D^{21}$ +64,2 ° (C = 1,13 g/100 ml Ethanol) erhalten. Das Isomer wird als "Verbindung 2" bezeichnet.
Rf = 0,22 (Iatroscan; Silicagel; Chloroform:Ethylacetat:n-Hexan = 4:1:5)
Bedingungen der chromatographischen Operation:
Innendurchmesser: 30 mm; Länge: 500 mm;
Stickstoff-Gas-Druck: 0,98 bar (1,0 kg/cm$^2$);
"Wako"-Gel (c-300): 150 g;
Elutionsmittel:
n-Hexan:Ethylacetat:Chloroform = 8:1:1.

Analog der oben beschriebenen Methode wird das R(+)-4-Chloro-α-methylbenzylamid der (±)-1,3,3-Trimethyl-2,2-dichlorocyclopropancarbonsäure behandelt, wodurch das entsprechende Diastereomer erhalten wird.

Die Strukturen und die Eigenschaften der erfindungsgemäß hergestellten Verbindungen 1 und 2 sind in Tabelle 1 zusammengestellt.

## Tabelle 1

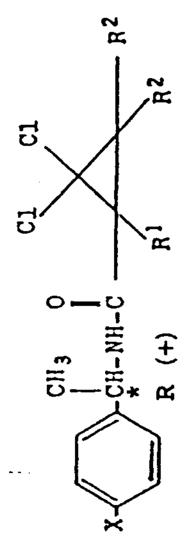

| Verbindung | X | $R^1$ | $R^2$ | Stereochemische Konfiguration | Spezifische Drehung [α] | Schmp. °C |
|---|---|---|---|---|---|---|
| 1 | Br | $CH_3$ | $CH_3$ | Amin-Struktureinheit: R(+) Säure-Struktureinheit: (±)-Epimere | $[\alpha]_D^{21}$ +61,2° (c=1,02 g/100 ml Ethanol) | 148 - 159 |
| 2 | Br | $CH_3$ | $CH_3$ | Amin-Struktureinheit: R(+)-Diastereomer | $[\alpha]_D^{21}$ +64,2° (c=1,13 g/100 ml Ethanol) | 147 - 148 |

Biotest-Beispiel

Bekannte Vergleichsverbindung A-1:

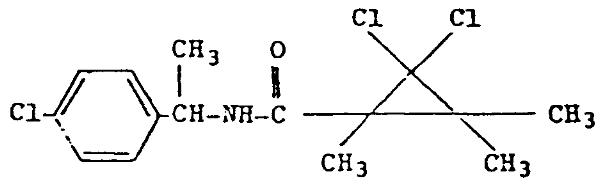

(gemäß der JP-OS 15867/1986).

Beispiel 4

Test gegen die Brusone-Krankheit bei Reis - Behandlung der Blätter

Formulierung der aktiven Verbindungen

Aktive Verbindung: 50 Gew.-Teile
Träger: Mischung aus Diatomeenerde und Kaolin (1:5) 45 Gew.-Teile
Emulgator: Polyoxyethylenalkylphenylether 5 Gew.-Teile

Zur Herstellung eines geeigneten Präparats der aktiven Verbindung wurde die angegebene Menge der aktiven Verbindung mit der angegebenen Menge Träger und der angegebenen Menge Emulgator vermischt, und das erhaltene benetzbare Pulver wird mit Wasser auf die gewünschte Konzentration verdünnt.

Test-Verfahren

Reispflanzen (Varietät Asahi) werden in Porzellan-Töpfen mit einem Durchmesser von 12 cm gezogen. Die Reis-Pflanzen, im 3- oder 4-Blatt-Stadium, werden mit dem Präparat der aktiven Verbindung in einer Menge von 50 ml auf jeweils 3 Töpfe besprüht. Am nächsten Tag werden die Pflanzen zweimal mit einer wäßrigen Sporen-Suspension des Brusone-Pilzes (Piricularia oryzae), der künstlich kultiviert worden ist, inokuliert und zwei Tage in eine Feuchtigkeitskammer von 25 °C und einer relativen Luftfeuchtigkeit von 100 % gestellt.

7 Tage nach dem Inokulieren wird der Infektionsgrad der Reispflanzen mittels der folgenden Skala bestimmt und aufgezeichnet.

| Grad der Infektion | Fläche (%), auf der die Pusteln auftreten |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

Der Grad des Pflanzenschutzes (%) wurde nach der Formel berechnet:

$$\text{Grad des Pflanzenschutzes (\%)} = \left[\frac{A - B}{A}\right] \times 100 ,$$

worin

A den Grad der Infektion in dem (unbehandelten) Kontrollabschnitt und
B den Grad der Infektion in dem behandelten Abschnitt bezeichnet.

In diesem Test bestand jeder Test-Abschnitt aus drei Töpfen. Die Ergebnisse sind in Tabelle 2 aufgeführt.

Tabelle 2

| Aktive Verbindung Nr. | Konzentration der aktiven Verbindung (ppm) | Grad der Schutzes (%) |
|---|---|---|
| 1 | 5<br>1 | 100<br>100 |
| 2 | 5<br>1 | 100<br>100 |
| (bekannt)<br>A-1 | 5<br>1 | 60<br>13 |

**Patentansprüche**

**1.** Optisch aktive Cyclopropancarboxamide der Formel (I)

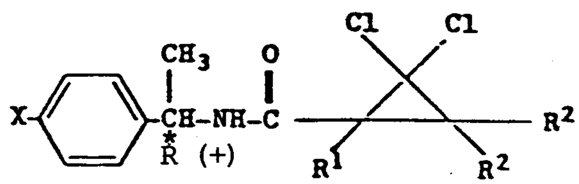

(I)

in der die Amin-Struktureinheit in der R(+)-Konfiguration vorliegt und in der

X Halogen bezeichnet,
$R^1$ für Wasserstoff und Alkyl steht und die
$R^2$s für Wasserstoff oder Alkyl stehen.

**2.** Verbindungen nach Anspruch 1, dadurch gekennzeichnet, daß

X Halogen ist und
$R^1$ für geradkettiges oder verzweigtes Alkyl mit 1 bis 6 Kohlenstoffatomen oder Wasserstoff steht und die
$R^2$s für geradkettiges oder verzweigtes Alkyl mit 1 bis 6 Kohlenstoffatomen oder Wasserstoff stehen.

**3.** Verbindungen nach Anspruch 1, dadurch gekennzeichnet, daß

X Chlor oder Brom bezeichnet,
$R^1$ einen geradkettigen oder verzweigten Alkyl-Rest mit 1 bis 4 Kohlenstoffatomen bezeichnet und die
$R^2$s Wasserstoff oder einen geradkettigen oder verzweigten Alkyl-Rest mit 1 bis 4 Kohlenstoffatomen bezeichnen.

**4.** Optisch aktive Verbindungen nach Ansprüchen 1 bis 3, dadurch gekennzeichnet, daß die Amin-Struktureinheit in der R(+)-Konfiguration vorliegt und die Säure-Struktureinheit in der racemischen Konfiguration vorliegt.

**5.** Optisch aktive Verbindungen nach Ansprüchen 1 bis 3, dadurch gekennzeichnet, daß die Verbindungen in der Form von Diastereoisomeren vorliegen, in denen die Amin-Struktureinheit die R(+)-Konfiguration hat.

**6.** Verfahren zur Herstellung optisch aktiver Cyclopropancarboxamide der Formel (I) gemäß Anspruch 1

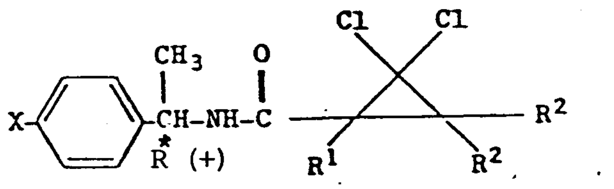

(I)

in welcher die Amin-Struktureinheit in der R( + )-Konfiguration vorliegt und in der

X Halogen bezeichnet,

$R^1$ für Wasserstoff oder Alkyl steht und die

$R^2$s für Wasserstoff oder Alkyl stehen,

dadurch gekennzeichnet, daß man

a) Verbindungen der Formel (II)

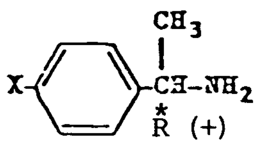

(II)

(in der X die oben angegebene Bedeutung hat) mit Verbindungen der Formel (III)

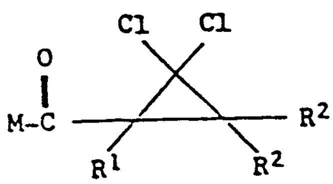

(III)

(in der $R^1$ und $R^2$ die oben angegebenen Bedeutungen haben und M für Hydroxyl oder ein Halogen steht), in Gegenwart eines inerten Lösungsmittels und gegebenenfalls in Gegenwart eines säurebindenden Mittels umgesetzt werden oder

optisch aktive Verbindungen der Formel (I), bei denen die Amin-Struktureinheit optisch aktiv ist, einer physikalischen Behandlung unterzogen werden.

**7.** Fungizide Mittel für die Landwirtschaft, dadurch gekennzeichnet, daß sie wenigstens eine Verbindung der Formel (I) nach den Ansprüchen 1 und 6 enthalten.

**8.** Verfahren zur Bekämpfung von Pflanzenkrankheiten, dadurch gekennzeichnet, daß man Verbindungen der Formel (I) nach den Ansprüchen 1 und 6 auf die Pathogene und/oder den Ort ihres Auftretens und den Ort des Auftretens der Pflanzenkrankheiten einwirken läßt.

**9.** Verwendung der Verbindungen der Formel (I) nach den Ansprüchen 1 und 6, zur Bekämpfung von Pflanzenkrankheiten.

**10.** Verfahren zur Herstellung von fungiziden Mitteln für die Landwirtschaft, dadurch gekennzeichnet, daß Verbindungen der Formel (I) nach den Ansprüchen 1 und 6 mit Streckmitteln und/oder grenzflächenaktiven Mitteln vermischt werden.

## Claims

**1.** Optically active cyclopropanecarboxamides of the formula (I)

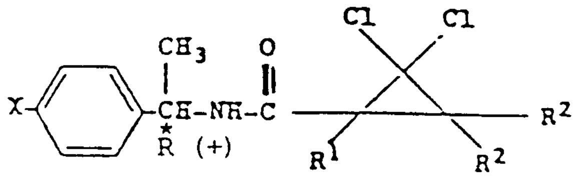

(I)

in which the amine structural unit is in the R(+) configuration and in which

X denotes halogen,

$R^1$ represents hydrogen and alkyl and the

$R^2$ radicals represent hydrogen or alkyl.

**2.** Compounds according to Claim 1, characterised in that

X is halogen and

$R^1$ represents straight-chain or branched alkyl having 1 to 6 carbon atoms or hydrogen and the

$R^2$ radicals represent straight-chain or branched alkyl having 1 to 6 carbon atoms or hydrogen.

**3.** Compounds according to Claim 1, characterised in that

X denotes chlorine or bromine,

$R^1$ denotes a straight-chain or branched alkyl radical having 1 to 4 carbon atoms and the

$R^2$ radicals denote hydrogen or a straight-chain or branched alkyl radical having 1 to 4 carbon atoms.

**4.** Optically active compounds according to Claims 1 to 3, characterised in that the amine structural unit is in the R(+) configuration and the acid structural unit is in the racemic configuration.

**5.** Optically active compounds according to Claims 1 to 3, characterised in that the compounds are in the form of diastereoisomers in which the amine structural unit is in the R(+) configuration.

**6.** Process for the preparation of optically active cyclopropanecarboxamides of the formula (I) according to Claim 1

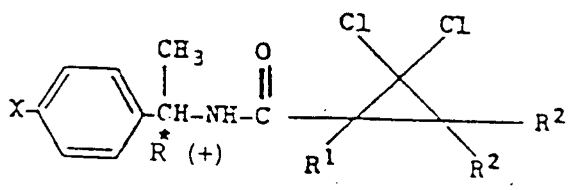

(I)

in which the amine structural unit is in the R(+) configuration and in which

X denotes halogen,

$R^1$ represents hydrogen or alkyl and the

$R^2$ radicals represent hydrogen or alkyl,

characterised in that

a) compounds of the formula (II)

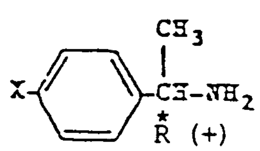

(II)

(in which X has the abovementioned meaning) are reacted with compounds of the formula (III)

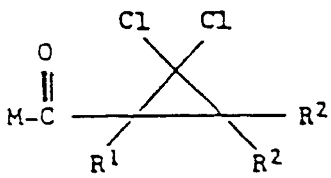

(III)

(in which $R^1$ and $R^2$ have the abovementioned meanings and M represents hydroxyl or a halogen), in the presence of an inert solvent and, if appropriate, in the presence of an acid-binding agent, or optically active compounds of the formula (I) in which the amine structural unit is optically active are subjected to a physical treatment.

**7.** Fungicidal agents for agriculture, characterised in that they contain at least one compound of the formula (I) according to Claims 1 and 6.

**8.** Method of combating plant diseases, characterised in that compounds of the formula (I) according to Claims 1 and 6 are allowed to act on the pathogens and/or the site where they occur and the site where the plant diseases occur.

**9.** Use of the compounds of the formula (I) according to Claims 1 and 6 for combating plant diseases.

**10.** Process for the preparation of fungicidal agents for agriculture, characterised in that compounds of the formula (I) according to Claims 1 and 6 are mixed with extenders and/or surface-active agents.

## Revendications

**1.** Cyclopropanecarboxamides optiquement actifs de formule (I)

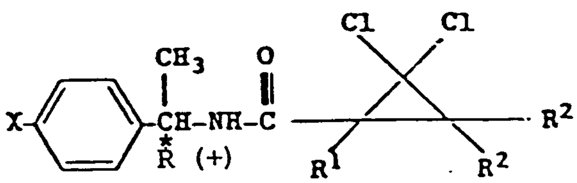

(I)

dans laquelle le motif structural amine se présente avec la configuration R(+) et dans laquelle

| | |
|---|---|
| X | désigne un halogène, |
| $R^1$ | représente l'hydrogène et un groupe alkyle et |
| les symboles $R^2$ | représentent de l'hydrogène ou des groupes alkyle. |

**2.** Composés suivant la revendication 1, caractérisés en ce que

| | |
|---|---|
| X | est un halogène, |
| $R^1$ | est un groupe alkyle à chaîne droite ou à chaîne ramifiée ayant 1 à 6 atomes de carbone ou l'hydrogène et |
| les symboles $R^2$ | représentent un groupe alkyle à chaîne droite ou à chaîne ramifiée ayant 1 à 6 atomes de carbone ou l'hydrogène. |

**3.** Composés suivant la revendication 1, caractérisés en ce que

| | |
|---|---|
| X | représente le chlore ou le brome, |
| $R^1$ | est un reste alkyle à chaîne droite ou à chaîne ramifiée ayant 1 à 4 atomes de carbone et |
| les symboles $R^2$ | représentent l'hydrogène ou un reste alkyle à chaîne droite ou à chaîne ramifiée ayant 1 à 4 atomes de carbone. |

**4.** Composés optiquement actifs suivant les revendications 1 à 3, caractérisés en ce que le motif structural amine se présente avec la configuration R(+) et le motif structural acide se présente avec la configuration racémique.

5. Composés optiquement actifs suivant les revendications 1 à 3, caractérisés en ce qu'ils se présentent sous la forme de diastéréo-isomères dans lesquels le motif structural amine a la configuration R( + ).

6. Procédé de production de cyclopropanecarboxamides optiquement actifs de formule (I) suivant la revendication 1

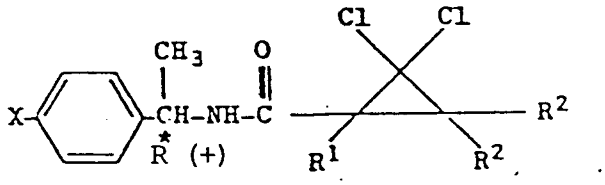

(I)

dans laquelle le motif structural amine se présente avec la configuration R( + ) et dans laquelle
X est un halogène,
$R^1$ est l'hydrogène ou un groupe alkyle et
les symboles $R^2$ représentent de l'hydrogène ou des groupes alkyle,
caractérisé en ce que
a) on fait réagir des composés de formule (II)

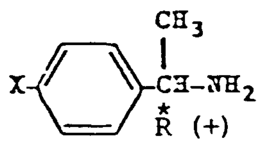

(II)

(dans laquelle X a la définition indiquée ci-dessus) avec des composés de formule (III)

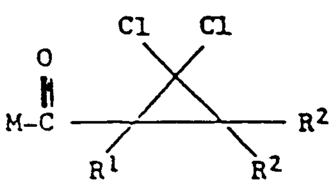

(III)

(dans laquelle $R^1$ et $R^2$ ont les définitions indiquées ci-dessus et M est un groupe hydroxyle ou un halogène) en présence d'un solvant inerte et, le cas échéant, en présence d'un accepteur d'acide, ou bien
b) on soumet des composés optiquement actifs de formule (I), dans lesquels le motif structural amine est optiquement actif, à un traitement physique.

7. Compositions fongicides pour l'agriculture, caractérisées en ce qu'elles contiennent au moins un composé de formule (I) suivant les revendications 1 et 6.

8. Procédé pour combattre des maladies des plantes, caractérisé en ce qu'on fait agir des composés de formule (I) suivant les revendications 1 et 6 sur les agents pathogènes et/ou sur leur lieu d'apparition et sur le lieu d'apparition des maladies des plantes.

9. Utilisation de composés de formule (I) suivant les revendications 1 et 6 pour combattre des maladies des plantes.

10. Procédé de préparation de compositions fongicides pour l'agriculture, caractérisé en ce qu'on mélange des composés de formule (I) suivant les revendications 1 et 6 avec des diluants et/ou des agents tensioactifs.